# FASCICULE DE BREVET EUROPEEN

(11) **EP 2 197 492 B1**
(45) Date de publication et mention de la délivrance du brevet: **23.11.2011**
(21) Numéro de dépôt: 08868434.5
(22) Date de dépôt: 09.10.2008
(51) Int. Cl.: A61K 47/24, A61K 47/44, A61K 9/00, A61K 9/107

(54) **NOUVELLES COMPOSITIONS A BASE DE TAXOIDES**
NEUARTIGE ZUSAMMENSETZUNGEN AUF TAXOIDBASIS
NOVEL TAXOID-BASED COMPOSITIONS

(30) Priorité: 10.10.2007 FR 0707092
(43) Date de publication de la demande: 23.06.2010
(73) Titulaire: Aventis Pharma S.A., 92160 Antony (FR)
(72) Inventeur: RORTAIS, Patricia, 75013 Paris (FR); GACHON, Carine, F-75013 Paris (FR)
(74) Mandataire: Gaslonde, Aude
(86) Numéro de dépôt international: PCT/FR2008/001410
(87) Numéro de publication internationale: WO 2009/083664

(56) Documents cités:
- EP-A- 1 498 120
- WO-A-2005/014048

## Description

La présente invention concerne une nouvelle forme pharmaceutique à base d'un agent thérapeutique ayant une activité antitumorale et antileucémique. Elle concerne plus particulièrement une nouvelle forme injectable contenant le dérivé de formule générale (I) suivante :

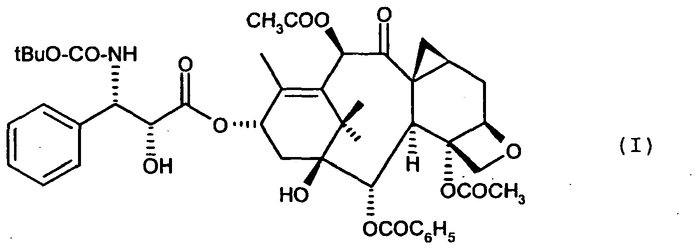

Ou le dérivé de formule (II) suivante :

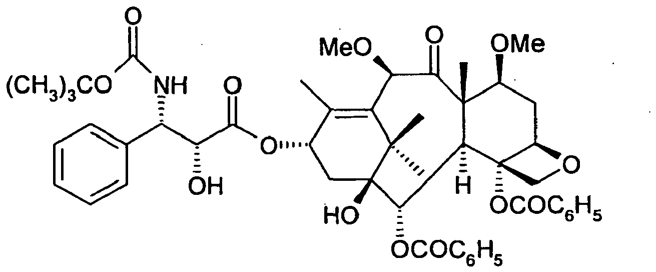

Le composé de formule (I) est plus connu sous la dénomination commune internationale larotaxel, il est actuellement en essais cliniques et connu sous l'abréviation XRP9881.Le composé de formule (II) est aussi actuellement en essais cliniques et connu sous la dénomination XRP6258.

Ces produits présentent in vivo une activité importante sur les tumeurs malignes ce qui a permis de les étudier dans le traitement des maladies résistantes à toutes les autres thérapies anticancéreuses.

Le document WO 2005/014048 montre des compositions orales auto-emulsionables contenant le composé de formule (I).

Malheureusement ce type de produit, notamment le docétaxel ou le paclitaxel, présente une solubilité dans l'eau tellement faible qu'il a été nécessaire de préparer une formulation pour préparation injectable à base d'agent tensioactif et d'éthanol. L'éthanol, est le meilleur solvant pharmaceutique qui permette de solubiliser les molécules répondant à la formule (I) comme à la formule (II). Une telle formulation est par exemple décrite dans le brevet EP 0 593 656 ou dans la publication suivante.

A titre d'exemple, selon la publication de Rowinsky, Lorraine, Cazenave et Donehower parue dans le Journal of the National Cancer Institute, vol. 82, No 15, pages 1247 à 1259, le 1er Août 1990, concernant le Taxol et qui présente une solubilité voisine des composés de formule (I) ou (II) on prépare une première solution, dite "solution mère", contenant environ 6 mg/mL de taxol dans un mélange solvant composé de:
- 50 % en volume d'éthanol
- 50 % en volume d'huile de ricin polyoxyéthyléné (ex Crémophor EL).

Toujours selon cette publication, pour obtenir des concentrations (entre 0,3 et 1 mg/mL) il est nécessaire d'injecter des solutions contenant en même temps que le principe actif des concentrations en chacun des composés suivants, éthanol et surtout Crémophor, d'environ 8 g pour 100 de solution de perfusion. Le traitement demandant souvent l'administration de doses élevées de principe actif et la concentration du principe actif dans la solution étant relativement faible l'injection de fort volume a pour effet de provoquer durant le traitement en plus des manifestations anaphylactiques des manifestations d'éthylisme.

Il a été découvert, selon le brevet européen EP 0 593 601, que la mise en oeuvre de formes pharmaceutiques différentes permettaient soit de diminuer fortement les concentrations en éthanol, soit encore de supprimer totalement le Crémophor et l'éthanol dans les solutés de perfusion.

Pour cela, on préparait une solution mère contenant le principe actif dans un mélange de solvants composé d'éthanol qui est le meilleur solvant biocompatible des principes actifs de la classe des taxanes et d'un agent tensioactif choisi parmi les polysorbates commercialisés notamment sous les dénominations Tween et Montanox, ou les ester-éther d'oxyde d'éthylène et de glycérides d'acides gras (huile de ricin hydrogénée ou non) commercialisés par exemple sous la dénomination de Crémophor ou d'Emulphor.

La solution mère, à faible teneur en éthanol, contient de préférence moins de 5 % d'éthanol, elle contient encore plus préférentiellement moins de 2 % d'éthanol. Cette solution est stable et peut ainsi contenir jusqu'à 200 mg/mL et de préférence jusqu'à 80 mg/mL de principe actif dans l'agent tensioactif.

La solution mère de paclitaxel présentait selon cette invention une concentration comprise entre 6 et 20 mg/ mL de principe actif dans l'agent tensioactif. La solution mère de docétaxel présentait de préférence une concentration comprise entre 20 et 80 mg/mL de principe actif dans l'agent tensioactif.

Ces solutions dans le tensioactif contenant éventuellement de faibles quantités d'éthanol pouvaient être dissoutes dans le soluté de perfusion mais moyennant une agitation extrêmement violente par exemple à l'aide d'un appareil type Vortex.

L'invention décrite dans le brevet EP 0 671 912 a permis de résoudre les problèmes laissés par le brevet précédent et consistait donc à réaliser une solution intermédiaire entre la solution des dérivés de la classe des taxanes dans le tensioactif et une solution aqueuse contenant un additif favorisant ultérieurement la mise en solution de la dite solution intermédiaire dans le soluté de perfusion.

Ces additifs étaient choisis parmi l'ensemble des additifs qui étaient capables de casser ou d'éviter la formation de la phase gélifiée qui est formée entre l'émulsifiant contenant le dérivé de la classe des taxanes et l'eau.

Parmi les additifs permettant de casser ou d'éviter la formation de cette phase gélifiée, étaient exemplifiés
- l'éthanol
- le glucose
- le glycérol
- le propylène glycol
- la glycine
- le sorbitol
- le mannitol
- l'alcool benzylique
- les polyéthylène glycols.

Les perfusions de docétaxel ou de paclitaxel sont ensuite injectées à l'homme à un débit prédéterminé en fonction de la quantité de principe actif que l'on veut injecter.

On observe avec toutes les formulations de l'art antérieur des phénomènes de chocs anaphylactiques plus ou moins modérés qui ont toujours été prévenus par l'administration préalable à la perfusion d'agent antihistaminique et/ou de corticoides.

Parmi les solutions récentes qui ont permis d'éviter l'apparition de ces manifestations anaphylactiques sont apparues des formulations où le taxoide est encapsulé dans une enveloppe à base d'albumine. Cette solution évite l'apparition des chocs anaphylactiques et donc le prétraitement avec des antihistaminique et/ou des corticoïdes et permet l'administration de doses plus fortes avec des effets secondaires réduits par rapport aux solutions de l'art antérieur. Cette formulation est par exemple décrite dans le brevet US 6,537,579.

La demande de brevet US2003/0099674 revendique des compositions lyophilisées constituées d'un taxoide solubilisé dans une goutte d'huile encapsulée par un agent tensioactif. Cette formulation lyophilisée ne collapse pas et ne se dégrade pas pendant le stockage. L'huile décrite contient des triglycérides à chaines moyennes (Miglyol 812N ou MCT) et l'huile de soja parmi de nombreuses autres huiles. Il est écrit page 7 [0072] que ces lyophilisats subissent moins de pertes et de détérioration que les émulsions liquides. Il est aussi mentionné [0089] que l'émulsion lors de sa préparation doit être soumise à la lyophilisation dans les heures ou minutes suivantes et lors de sa reconstitution le lyophilisat doit aussi être utilisé dans les heures ou minutes qui suivent.

Parmi les exemples sont décrits table 2 des solutions de paclitaxel dans l'huile avec de la lécithine, leur stabilité à un mois à TA sont bonnes pour le Miglyol et l'huile de soja. Une solution contenant de l'eau avec de l'huile de sésame est aussi décrite.

Parmi les émulsions obtenues avec mise en solution du lyophilisat ne sont décrites que des stabilités réalisées à -20°C sur 1 mois. Ces conditions de stabilité ne présentent aucun avantage par rapport aux solutions décrites dans les premiers brevets en ce qui concerne leur stabilité, notre objectif étant l'obtention de formulations prêtes à l'emploi et stables pendant au moins un an à température ambiante.

Au jour d'aujourd'hui on est toujours à la recherche de formulations permettant d'administrer des doses suffisantes d'agents de la classe des taxoides en évitant d'utiliser les agents tensioactifs du type huile de ricin polyoxyéthyléné ou polysorbates et dans lesquelles le taxoide est stable à température ambiante et se présente sous une forme liquide permettant un mélange avec le soluté de perfusion sans apparition de phénomène physique entraînant la séparation des phases c'est à dire soit la précipitation soit la séparation de phases liquides. On cherche en plus des formulations permettant d'administrer le taxoide en bolus en évitant les perfusions.

En plus la formulation recherchée doit permettre de conserver le produit stable à température ambiante au cours du temps et doit éviter la dégradation du taxoide par exemple par hydrolyse.

La présente invention répond à ces objectifs et concerne une émulsion à base d'une huile pharmaceutiquement acceptable dans l'eau en présence de lécithine ou de phospholipide d'hémisynthèse et contenant le taxoide.

Parmi les huiles pharmaceutiquement acceptables on peut citer l'huile de soja utilisée seule ou les triglycéride à chaînes moyennes. (C8-C10) tel que le Miglyol^{™} ou MCT^{™} utilisés seuls ou encore préférentiellement on peut utiliser un mélange d'huile de soja et MCT. Notamment le ratio pondéral entre l'huile de soja et le triglycéride est de 1 :1.

L'émulsion est formée par l'addition de lécithine de soja ou d'oeuf, de préférence d'oeuf telle que celle commercialisée sous la marque Lipoid E80, ou par addition préférentielle d'une phosphatidylcholine qui est un agent tensioactif zwiterionique.

L'émulsion présente une meilleure stabilité physique, lorsque la lécithine ou la phosphatidylcholine est associée à un phospholipide anionique choisi notamment parmi les phosphatidyl glycérols ou les acides phosphatidiques.

On peut citer parmi les phosphatidyl glycérols le dilauroylphosphatidyl glycérol (DLPG), le dimyristoylphosphatidyl glycérol (DMPG) ou le dipalmytoylphosphatidyl glycérol (DPPG). On peut citer parmi les acides phosphatidiques les sels de sodium de l'acide dilauroyl glycero phosphatidique (DLPA), de l'acide dimyristoyl glycero phosphatidique (DMPA) ou de l'acide dipalmytoyl glycero phosphatidique (DPPA).

Le rapport pondéral entre respectivement la lécithine ou la phosphatidylcholine et le phosphatidyl glycérol ou l'acide phosphatidique est de préférence compris entre 500 et 3 et encore plus préférentiellement entre 100 et 10. On préfère parmi tous les phospholipides anioniques le dilauroylphosphatidyl glycérol.

Le pH de la formulation peut être ajusté entre 3 et 7 par addition d'acide. On utilise de préférence un tampon pour conserver le pH dans cette zone associé ou non à un acide. Le tampon est de préférence l'histidine. On préfère utiliser 5 à 50 mM d'histidine dans la solution. L'acide est choisi de préférence parmi l'acide chlorhydrique, citrique ou l'acide ascorbique.

L'isotonicité de la solution peut être ajustée par addition de glycérol.

On peut utiliser pour la facilité de mise en oeuvre de la formulation 0 à 2% d'éthanol.

De préférence, la composition pondérale de la formulation est comprise dans les limites suivantes :
- phase huileuse 10 à 30%
- lécithine ou phosphatidylcholine 0,6 à 5 %
- phosphatidylglycérol 0,01 à 0,2%
- glycérol 0 à 2.5%
- éthanol 0 à 2%
- eau qsp 100%

La préparation de l'émulsion est de préférence réalisée à l'aide d'un appareil de microfluidisation, ou un homogénéiseur haute pression de façon à obtenir des gouttelettes d'une taille comprise entre 100 nm et 1 µm et de préférence entre 100 et 500 nm. On préfère encore plus particulièrement les formulations où la taille moyenne des gouttelettes est comprise entre 150 et 350 nm. La formulation finale est inertée de préférence par de l'azote ou par un gaz inerte ne contenant pas d'oxygène.

Les concentrations en principe actif de formule (I) ou de formule (II) sont inférieures à 10mg/mL et sont notamment utilisées selon une concentration comprise entre 0,5 et 6 mg/mL et de préférence d'environ 4 mg/mL.

L'invention sera plus complètement décrite à l'aide des exemples suivants qui ne doivent pas être considérés comme limitatifs de l'invention.

### EXEMPLE 1 :

1 g de DPPC et 0.03 g de DLPG sont solubilisés à 60° C dans le mélange MCT (10 g) huile de soja (10 g) et éthanol (0.4 g).
0.4 g du composé de formule (I) sont mis en solution dans la solution lipidique précédemment obtenue.
2.5 g de glycérol sont ajoutés sous agitation à 75.7 g d'un tampon aqueux 10 mM d'histidine jusqu'à obtention d'un mélange homogène

La phase aqueuse est ajoutée à la phase huileuse et l'ensemble est homogénéisé au broyeur (Ultraturrax) avant passage sur Microfluidiseur (11 cycles à 11000 PSI). La taille des gouttelettes obtenues est d'environ 250nm.

### EXEMPLE 2 :

1 g de DPPC et 0.03 g de DLPG sont solubilisés à 60° C dans le mélange MCT (10 g) huile de soja (10 g) et éthanol (2 g).
0.4 g de composé de formule (I) sont mis en solution dans la solution lipidique précédemment obtenue.
2.5 g de glycérol sont ajoutés sous agitation à 75.7 g d'un tampon aqueux 10 mM d'histidine ajusté à pH 6 (HCl) jusqu'à obtention d'un mélange homogène

La phase aqueuse est ajoutée à la phase huileuse et l'ensemble est homogénéisé à l'Ultraturrax avant passage sur Microfluidiseur (11 cycles à 11000 PSI). La taille des gouttelettes obtenues est d'environ 280nm.

### EXEMPLE 3 :

1.2 g de Lipoïd E80 et 0.03 g de DLPG sont solubilisés à 60° C dans le mélange MCT (10 g) huile de soja (10 g).
0.4 g du composé de formule (I XRP9881) sont mis en solution dans la solution lipidique précédemment obtenue.
2.5 g de glycérol sont ajoutés sous agitation à 75.7 g d'un tampon aqueux 10 mM d'histidine jusqu'à obtention d'un mélange homogène.

La phase aqueuse est ajoutée à la phase huileuse et l'ensemble est homogénéisé à l'Ultraturrax avant passage sur Microfluidiseur (11 cycles à 11000 PSI). La taille moyenne des gouttelettes obtenues est d'environ 140 nm (Dv 50).

Stabilité physique 1 mois 60°C : tailles inchangées (Dv 50 et Dv90).

### EXEMPLE 4 :

1.2 g de Lipoïd E80 et 0.03 g de DLPG sont solubilisés à 60° C dans le mélange MCT (10 g) huile de soja (10 g).
0.4 g du composé de formule (I XRP9881) sont mis en solution dans la solution lipidique précédemment obtenue.

2.5 g de glycérol sont ajoutés sous agitation à 75.7 g d'un tampon aqueux 10 mM d'histidine ajusté à pH 6 (HCl) jusqu'à obtention d'un mélange homogène.

La phase aqueuse est ajoutée à la phase huileuse et l'ensemble est homogénéisé à l'Ultraturrax avant passage sur Microfluidiseur (11 cycles 11000 PSI ). La taille moyenne des gouttelettes obtenues est d'environ 140 nm (Dv 50) .

Stabilité physique 1 mois 60°C : tailles inchangées (Dv50 et Dv90).

### EXEMPLE 5

1 g de DPPC et 0.03 g de DLPG sont solubilisés à 60° C dans le mélange MCT (10 g) huile de soja (10 g) et éthanol (0.4 g).
0.4 g du composé de formule (I XRP9881) sont mis en solution dans la solution lipidique précédemment obtenue.

2.5 g de glycérol sont ajoutés sous agitation à 75.7 g d'un tampon aqueux 10 mM d'histidine ajusté à pH6 (HCl) jusqu'à obtention d'un mélange homogène.

La phase aqueuse est ajoutée à la phase huileuse et l'ensemble est homogénéisé à l'Ultraturrax avant passage sur Microfluidiseur (11 cycles à 11000 PSI).

La taille moyenne des gouttelettes obtenues est d'environ 280 nm (Dv50).

Stabilité physique 1 mois 60C : tailles inchangées (Dv 50 et Dv 90).

### EXEMPLE 6 :

1.2% de Lipoïd E80 et 0.03 g de DMPG sont solubilisés à 60° C dans le mélange MCT (10 g) huile de soja (10 g).
0.4 g de composé de formule (I XRP9881) sont mis en solution dans la solution lipidique précédemment obtenue.
2.5 g de glycérol sont ajoutés sous agitation à 75.7 g d'eau PPI jusqu'à obtention d'un mélange homogène.

La phase aqueuse est ajoutée à la phase huileuse et l'ensemble est homogénéisé à l'Ultraturrax avant passage sur Microfluidiseur (11 cycles à 11000 PSI). La taille moyenne des gouttelettes obtenues est d'environ 140 nm (Dv50).

Stabilité physique 1 mois 60°C : tailles inchangées (Dv 50 et Dv90).

### EXEMPLE 7 :

1.2 g Lipoïd E80 et 0.03 g de DPPA sont solubilisés à 60° C dans le mélange MCT (10 g) huile de soja (10 g).
0.4 g de composé de formule (I XRP9881) sont mis en solution dans la solution lipidique précédemment obtenue.
2.5 g de glycérol sont ajoutés sous agitation à 75.7 g d'eau PPI jusqu'à obtention d'un mélange homogène.

La phase aqueuse est ajoutée à la phase huileuse et l'ensemble est homogénéisé à l'Ultraturrax avant passage sur Microfluidiseur (11 cycles 11000 PSI). La taille moyenne des gouttelettes obtenues est d'environ 140 nm (Dv 50).

Stabilité physique 1 mois 60°C : tailles inchangées (Dv 50 et Dv90).

### EXEMPLE 8

1 g de DPPC et 0.03 g de DLPG sont solubilisés à 60° C dans le mélange MCT (10 g) huile de soja (10 g) et éthanol (0.4 g).
0.4 g du composé de formule (II XRP6258) sont mis en solution dans la solution lipidique précédemment obtenue.
2.5 g de glycérol sont ajoutés sous agitation à 75.7 g d'un tampon aqueux 10 mM d'histidine ajusté à pH6 (HCl) jusqu'à obtention d'un mélange homogène.

La phase aqueuse est ajoutée à la phase huileuse et l'ensemble est homogénéisé à l'Ultraturrax avant passage sur Microfluidiseur (11 cycles à 11000 PSI).

La taille moyenne des gouttelettes obtenues est d'environ 260 nm.

### EXEMPLE COMPARATIF

L'exemple suivant a été réalisé en comparaison avec la demande de brevet US 2003/0099674 pour démontrer que la lyophilisation est une technologie qui ne peut s'appliquer aux formulations de l'invention car elle provoque un agrandissement des particules lipidiques d'invention.

### 1. MATERIEL ET METHODES

### 1.1.matériel

Lipoid E80 - lot 1031471-7/906 : fournisseur : LIPOÏD KG.
Miglyol 812
Maltose monohydrate.

Saccharose.

### 1.2. Formule

| Formule | % | Unitaire (g) | |
|---|---|---|---|
| XRP9881* | 0,340 | 0,002 | 0,007 |
| Miglyol 812 | 17,0 | 0,085 | 0,340 |
| Lipoid E80 | 1,0 | 0, 005 | 0,020 |
| Eau purifiée | 66,6 | 0,333 | 1,333 |
| Cryoprotecteur | 15.0 | 0.075 | 0.300 |
| Total | 100 | 0,5 | 2 |
| Hauteur lyophilisat | | 1mm | 6mm |

| | | | |
|---|---|---|---|
| * produit pur et exempt de solvant | | | |

### 1.3. Mode de préparation des émulsions

1) Solubilisation du principe actif dans l'huile.
2) Pré-dispersion à l'ultra-turrax de la phase huileuse dans l'eau + lécithine.
3) Homogénéisation sur microfluidiseur à 11000 PSI (11 passages.
4) Division de l'émulsion en 2 fractions - Addition de 15% de maltose ou de saccharose.
5) Répartition QS 1 mm et 6 mm de hauteur.
6) Lyophilisation : Les conditions appliquées sont décrites ci-dessous, Congélation : température étagère -45°C - température produit -39°C.

Sublimation : température étagère -25°C - pression 50 microbar ?
Dessiccation secondaire à 30°C.

### 2. RESULTAT

### 2.1. Avant lyophilisation

Taille avant ajout des cryoprotecteurs 250 nm (Coulter N4+, méthode de mesure : diffusion quasi élastique de la lumière).

Pas de modification de taille après ajout des cryoprotecteurs (avant lyophilisation).

### 2.2. Après lyophilisation

### 2.2.1. Aspect et reconstitution

Aspect du lyophilisat : pas de collapse ni de rétraction → aspect correct.

Reconstitution avec eau PPI : reconstitution immédiate, l'émulsion obtenue est d'aspect homogène.

### 2.2.2. Taille après reconstitution

Présence de vésicules de taille comprise entre 300 nm et 10 µm (système métastable).

Pas de différence visible ou mesurée entre maltose et saccharose, ni entre 1 et 6 mm de hauteur de lyophilisat.

## Revendications

1. Compositions injectables sous forme d'émulsion contenant des dérivés de la classe des taxanes composées d'une émulsion à base d'une huile pharmaceutiquement acceptable dans l'eau et de lécithine composition dans laquelle le taxane est dissous et répond aux formules générales (I) ou (II) suivante : 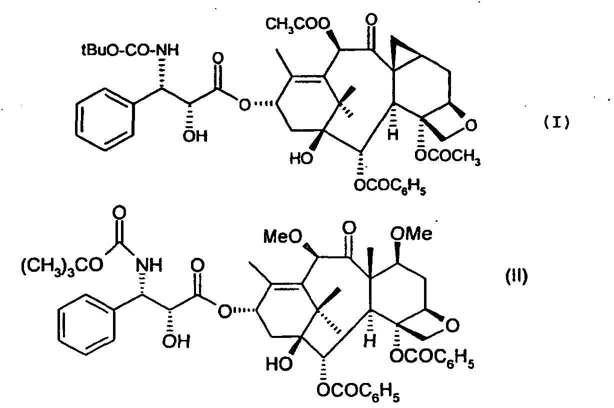 **caractérisées en ce qu'**elle contiennent en plus un agent tensioactif anionique.

2. Compositions selon la revendication 1 **caractérisées en ce que** les huiles pharmaceutiquement acceptables sont choisies parmi les mélanges d'huile de soja et de triglycérides à chaines moyennes.

3. Compositions selon la revendication 1 **caractérisées en ce que** la lécithine est une lécithine naturelle choisie parmi les lécithine d'oeuf ou de soja ou d'hémisynthèse choisie parmi les phosphatidylcholines.

4. Compositions selon la revendication 1 **caractérisées en ce que** l'agent tensioactif anionique est un phosphatidylglycérol ou un acide phosphatidique.

5. Compositions selon la revendication 4 **caractérisées en ce que** le phosphatidylglycérol est choisi parmi le dilauroylphosphatidyl glycérol (DLPG), le dimyristoylphosphatidyl glycérol (DMPG) ou le dipalmytoylphosphatidyl glycérol (DPPG).

6. Compositions selon la revendication 4 **caractérisées en ce que** l'acide phosphatidique est choisi parmi les sels de sodium de l'acide dilauroyl glycero phosphatidique DLPA), de l'acide dimyristoyl glycero phosphatidique (DMPA) ou de l'acide dipalmytoyl glycero phosphatidique (DPPA).

7. Compositions selon la revendication 1 **caractérisées en ce que** le rapport pondéral entre la phosphatidylcholine ou la lécithine et le phosphatidyl glycérol ou l'acide phosphatidique est de préférence compris entre 500 et 3 .

8. Compositions selon la revendication 7 **caractérisée en ce que** le rapport pondéral est de préférence compris entre 100 et 10.

9. Compositions selon la revendication 1 **caractérisées en ce que** le pH de la formulation est ajusté entre 3 et 7 par addition d'acide.

10. Compositions selon la revendication 9 **caractérisées en ce que** l'acide est choisi parmi l'acide chlorhydrique, citrique ou l'acide ascorbique.

11. Compositions selon la revendication 1 **caractérisée en ce qu'**on ajoute un tampon qui est l'histidine.

12. Compositions selon la revendication 1 **caractérisées en ce que** l'isotonicité de la solution est ajustée par addition de glycérol.

13. Compositions selon la revendication 1 **caractérisées en ce que** la composition pondérale de la formulation est comprise dans les limites suivantes :
- phase huileuse 10 à 30%
- lécithine ou phosphatidylcholine 0,6 à 5 %
- phosphatidylglycérol 0,01 à 0,2%
- glycérol 0 à 2.5%
- éthanol 0 à 2%
- eau qsp 100%

14. Compositions selon l'une quelconque des revendications précédentes **caractérisées en ce qu'**elles sont administrées en bolus par voie intraveineuse.

15. Compositions selon l'une quelconque des revendications précédentes **caractérisées en ce que** les particules ont une taille comprise entre 100nm à 1 µm.

16. Procédé de préparation des compositions selon l'une quelconque des revendications précédentes **caractérisé en ce que** l'émulsion est réalisée à l'aide d'un appareil de microfluidisation ou d'un homogénéiseur haute pression.

## Claims

1. Compositions that can be injected in the form of an emulsion containing derivatives of the taxane class, composed of an emulsion based on a pharmaceutically acceptable oil in water and on lecithin, composition in which the taxane is dissolved and corresponds to the general formula (I) or (II) below: 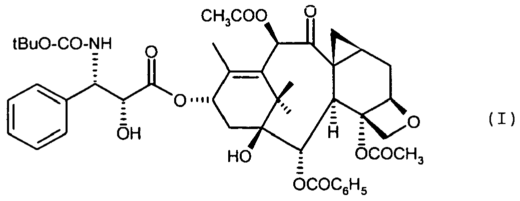 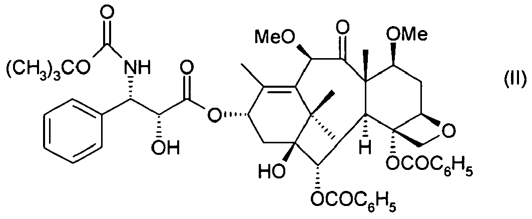 **characterized in that** they also contain an anionic surfactant.

2. Compositions according to Claim 1, **characterized in that** the pharmaceutically acceptable oils are chosen from mixtures of soybean oil and of medium-chain triglycerides.

3. Compositions according to Claim 1, **characterized in that** the lecithin is a natural lecithin chosen from egg lecithin or soy lecithin or a semi-synthetic lecithin chosen from phosphatidylcholines.

4. Compositions according to Claim 1, **characterized in that** the anionic surfactant is a phosphatidylglycerol or a phosphatidic acid.

5. Compositions according to Claim 4, **characterized in that** the phosphatidylglycerol is chosen from dilauroylphosphatidylglycerol (DLPG), dimyristoyl-phosphatidylglycerol (DMPG) or dipalmitoylphosphatidyl-glycerol (DPPG).

6. Compositions according to Claim 4, **characterized in that** the phosphatidic acid is chosen from the sodium salts of dilauroylglycerophosphatidic acid (DLPA), of dimyristoylglycerophosphatidic acid (DMPA) or of dipalmitoylglycerophosphatidic acid (DPPA).

7. Compositions according to Claim 1, **characterized in that** the weight ratio of the phosphatidylcholine or of the lecithin to the phosphatidylglycerol or to the phosphatidic acid is preferably between 500 and 3.

8. Compositions according to Claim 7, **characterized in that** the weight ratio is preferably between 100 and 10.

9. Compositions according to Claim 1, **characterized in that** the pH of the formulation is adjusted to between 3 and 7 by addition of acid.

10. Compositions according to Claim 9, **characterized in that** the acid is chosen from hydrochloric acid, citric acid or ascorbic acid.

11. Compositions according to Claim 1, **characterized in that** a buffer, which is histidine, is added.

12. Compositions according to Claim 1, **characterized in that** the isotonicity of the solution is adjusted by addition of glycerol.

13. Compositions according to Claim 1, **characterized in that** the weight composition of the formulation is within the following limits:
- oily phase 10 to 30%
- lecithin or phosphatidylcholine 0.6 to 5%
- phosphatidylglycerol 0.01 to 0.2%
- glycerol 0 to 2.5%
- ethanol 0 to 2%
- water qs for 100%

14. Compositions according to any one of the preceding claims, **characterized in that** they are administered intravenously as a bolus.

15. Compositions according to any one of the preceding claims, **characterized in that** the particles have a size between 100 nm and 1 µm.

16. Method of preparing compositions according to any one of the preceding claims, **characterized in that** the emulsion is produced using a microfluidization apparatus or a high-pressure homogenizer.

## Patentansprüche

1. Injizierbare Zusammensetzungen in Form einer Emulsion, die Derivate der Taxanklasse enthält, bestehend aus einer Emulsion auf Basis eines pharmazeutisch unbedenklichen Öls in Wasser und aus einer Lecithinzusammensetzung, in der das Taxan, das den folgenden allgemeinen Formeln (I) oder (II) entspricht: 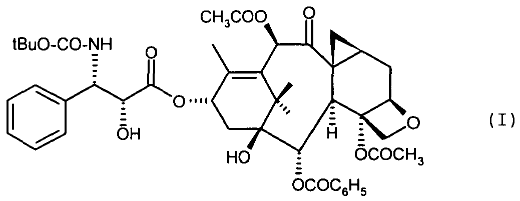 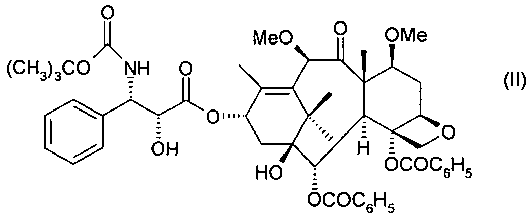 gelöst ist, **dadurch gekennzeichnet, dass** sie mehr als ein anionisches Tensid enthalten.

2. Zusammensetzungen nach Anspruch 1, **dadurch gekennzeichnet, dass** die pharmazeutisch unbedenklichen Öle aus den Mischungen aus Sojaöl und mittelkettigen Triglyceriden stammen.

3. Zusammensetzungen nach Anspruch 1, **dadurch gekennzeichnet, dass** es sich bei dem Lecithin um ein natürliches Lecithin aus der Reihe Eilecithin oder Sojalecithin oder um ein halbsynthetisches Lecithin aus der Reihe der Phosphatidylcholine handelt.

4. Zusammensetzungen nach Anspruch 1, **dadurch gekennzeichnet, dass** es sich bei dem anionischen Tensid um Phosphatidylglycerin oder eine Phosphatidsäure handelt.

5. Zusammensetzungen nach Anspruch 4, **dadurch gekennzeichnet, dass** das Phosphatidylglycerin aus der Reihe Dilauroylphosphatidylglycerin (DLPG), Dimyristoylphosphatidylglycerin (DMPG) oder Dipalmitoylphosphatidylglycerin (DPPG) stammt.

6. Zusammensetzungen nach Anspruch 4, **dadurch gekennzeichnet, dass** die Phosphatidsäure aus der Reihe der Natriumsalze der Dilauroylglycerophosphatidsäure (DLPA), Dimyristoylglycerophosphatidsäure (DMPA) oder Dipalmitoylglycerophosphatidsäure (DPPA) stammt.

7. Zusammensetzungen nach Anspruch 1, **dadurch gekennzeichnet, dass** das Gewichtsverhältnis zwischen Phosphatidylcholin oder Lecithin und Phosphatidylglycerin oder Phosphatidsäure vorzugsweise zwischen 500 und 3 beträgt.

8. Zusammensetzungen nach Anspruch 7, **dadurch gekennzeichnet, dass** das Gewichtsverhältnis vorzugsweise zwischen 100 und 10 liegt.

9. Zusammensetzungen nach Anspruch 1, **dadurch gekennzeichnet, dass** der pH der Formulierung durch Versetzen mit Säure zwischen 3 und 7 eingestellt wird.

10. Zusammensetzungen nach Anspruch 9, **dadurch gekennzeichnet, dass** die Säure aus der Reihe Chlorwasserstoffsäure, Citronensäure und Ascorbinsäure stammt .

11. Zusammensetzungen nach Anspruch 1, **dadurch gekennzeichnet, dass** man mit einem Puffer, bei dem es sich um Histidin handelt, versetzt.

12. Zusammensetzungen nach Anspruch 1, **dadurch gekennzeichnet, dass** die Isotonizität der Lösung durch Zugabe von Glycerin eingestellt wird.

13. Zusammensetzungen nach Anspruch 1, **dadurch gekennzeichnet, dass** die Gewichtszusammensetzung der Formulierung innerhalb der folgenden Grenzen liegt:
- Ölphase 10 bis 30%
- Lecithin oder Phosphatidylcholin 0,6 bis 5%
- Phosphatidylglycerin 0,01 bis 0,2%
- Glycerin 0 bis 2,5%
- Ethanol 0 bis 2%
- Wasser ad 100%

14. Zusammensetzungen nach einem der vorhergehenden Ansprüche, **dadurch gekennzeichnet, dass** sie intravenös als Bolus verabreicht werden.

15. Zusammensetzungen nach einem der vorhergehenden Ansprüche, **dadurch gekennzeichnet, dass** die Partikel eine Größe zwischen 100 nm und 1 µm aufweisen.

16. Verfahren zur Herstellung von Zusammensetzungen nach einem der vorhergehenden Ansprüche, **dadurch gekennzeichnet, dass** die Emulsion mit Hilfe eines Mikrofluidisierungsapparats oder eines Hochdruckhomogenisators hergestellt wird.
